## Europäisches Patentamt

## European Patent Office

## Office européen des brevets

(11) Publication number: **0 127 737 B1**

(12) **EUROPEAN PATENT SPECIFICATION**

(45) Date of publication of patent specification: **11.05.88**

(51) Int. Cl.⁴: **C 07 K 1/14,** C 07 K 3/28, A 61 K 37/02, B 01 D 13/00

(21) Application number: **84103080.2**

(22) Date of filing: **21.03.84**

(54) A process for the recovery of a peptide-containing compound.

(30) Priority: **09.05.83 SE 8302638**

(43) Date of publication of application:
**12.12.84 Bulletin 84/50**

(45) Publication of the grant of the patent:
**11.05.88 Bulletin 88/19**

(84) Designated Contracting States:
**AT BE CH DE FR GB IT LI LU NL**

(56) References cited:
**EP-A-0 046 915**
**FR-A-2 330 694**
**US-A-3 843 444**

(73) Proprietor: **Gambro Lundia AB**
**Box 10101**
**S-220 10 Lund (SE)**

(72) Inventor: **Nylen, Ulf Thomas Gustav**
**Borgarevägen 15**
**S-222 47 Lund (SE)**
Inventor: **Mattiasson, Bo Gustav**
**Trädgardsmästaren 26**
**S-222 48 Lund (SE)**

(74) Representative: **Boberg, Nils Gunnar Erik et al**
**Gambro AB Patent Department Box 10101**
**S-220 10 Lund (SE)**

Courier Press, Leamington Spa, England.

## Description

### Technical field

The invention relates to a process for the recovery of a peptide-containing compound from a solution also containing one or more other compounds of contaminating character, this process comprising the steps of

a) mixing a solution of the said peptide containing compound and the said compounds of contaminating character with a ligand bound to a carrier in order to selectively form a complex consisting of carrier/ligand/peptide containing compound,

b) subjecting the reaction mixture of step (a) to a membrane filtration in order to separate the complex formed in step (a) from the contaminating compounds,

c) splitting of the compound to be recovered from the said complex formed in step (a) and

d) separating the compound from the ligand bound to the carrier by membrane-filtration.

The invention relates in particular to a process of the above mentioned type for the recovery of enzymes, lectins, antibodies, antigens and haptens, suitable examples of applicable ligands bound to carriers being inhibitors, carbohydrate structures, antigens, antibodies, haptens, cofactors, complement factors (e.g. C1Q), enzymes, nucleic acids, conglutinin and surface receptors, for example bacterial, such as protein A and protein G. The invention in its widest scope can also be used, moreover, for the recovery of types of compounds other than those enumerated above including charged compounds of the ionic type.

### Background art

A process of the type described is known from the European patent application bearing publication number 0 046 915. It is a disadvantage of this known process that it makes use of a carrier in dissolved form which, owing to its elongated molecular structure, may slip through the pores of the membrane used. Theoretically this problem should be avoidable by using a membrane with a lower top cut-off limit, but in practice it is difficult to provide such a "tailor-made" membrane, so that the problem at most can be reduced, but not totally and completely eliminated. In addition a membrane which has such a lower top cut-off limit, i.e. within the interval appropriate in this particular context smaller than $1 \times 10^6$ Dalton, requires a complicated technique of manufacture and is therefore considerably more expensive than the more readily accessible membranes with a top cut-off limit around $1 \times 10^6$ Dalton or over, e.g. up to $100 \times 10^6$ Dalton.

It is an object of the invention, therefore, to provide an improved process which utilizes the known technique of combined affinity-chromatographic purification and ultrafiltration, but which also makes possible the use of the aforementioned less expensive membranes without any risk of leakage of the carrier used.

The objects are achieved in accordance with the invention by the process which is defined in the claims following hereafter and which will be described in more detail in the following.

### Disclosure of invention

In accordance with the invention a process of the type described in the introduction is thus provided for the recovery of a peptide-containing compound. The process is characterized in that the carrier is constituted of a macromolecular solid material and that the peptide-containing compound is separated by using a semipermeable membrane which is permeable for the peptide-containing compound, but impermeable for the macromolecular solid material with bound ligand.

A macromolecular solid material in accordance with the invention, as distinguished from the elongated soluble carriers according to the known technique, has a more compact shape which through suitable dimensioning can be made sufficiently large in order to prevent passage through the pores of the membrane used even if the latter has a top cut-off limit around $1 \times 10^6$ Dalton or over, for example up to $100 \times 10^6$ Dalton.

Examples of suitable solid macromolecular material in accordance with the invention are cells, agarose, starch, cellulose, chitin, gelatine, collagen and synthetic polymers of the latex and acrylic derivative type, as well as inorganic materials, for example ceramic material, porous siliceous material etc. The solid material is used preferably in the form of spherical particles of a sufficiently large diameter so that they cannot penetrate through the pores of a membrane with a top cut-off limit around $1 \times 10^6$ Dalton or over, for example up to $100 \times 10^6$ Dalton. Examples of such diameters are over 0.1 μm, such as between 1 and 50 μm, preferably 2—10 μm.

As mentioned earlier, the ligand which is to be bound to the macromolecular solid material may be selected among inhibitors, carbohydrate structures, antigens, antibodies, haptens, complement factors (e.g. C1Q), cofactors, enzymes, nucleic acis, conglutinin and surface receptors, for example bacterial, such as protein A and protein G, depending upon the type of peptide-containing compound which is to be recovered. One important demand which is made on the ligand is that it ought to be able to bind with biospecific affinity to the desired peptide-containing compound.

The expression "bound to the solid macromolecular material" refers to any suitable type of binding, e.g. physical or chemical binding. Moreover, it also includes such cases where the ligand is naturally occurring on the solid material, e.g. surface structures on killed microbial cells, for example yeast cells.

In accordance with the invention it is appropriate to separate the impurities from the solution containing the complex of carrier/ligand/peptide-containing compound formed by using a semipermeable membrane which is permeable for them, but impermeable for the complex formed.

Examples of suitable membranes for this purification are those which have a cut-off of between 50 000 and $100 \times 10^6$ Dalton, where the lower limit, of course, can be higher depending on the size of the impurities. Generally it can be said that the impurities will be separated the more rapidly, the higher the bottom cut-off limit. In accordance with the invention the peptide-containing compound may be split off from the complex formed in any appropriate manner. It may be achieved, for example, through modification of the pH of the solution, by the addition, for example, of an acid, or through modification of the ionic strength of the solution, by the addition, for example, of a salt solution. The splitting off can also be achieved through the addition of a ligand present in free form in a sufficient concentration to drive out the ligand bound to the carrier and thus break the bond between this and the peptide-containing compound. The choice of a suitable free ligand is determined in each individual case by the type of compound which is to be recovered, whilst it is a general rule that the ligand chosen should be able to bind to this compound.

Examples of such ligands which can be used are low-molecular sugars, e.g. mono and disaccharides in the purification of lectins and antigens or parts thereof in the purification of antibodies.

The peptide-containing compound is separated appropriately from the ligand added in free form by the use of a semipermeable membrane with a sufficiently low top cut-off limit to prevent penetration by the peptide-containing compound but with a sufficiently high top cut-off limit to allow passage of the free ligand, as will be explained further down. A semipermeable membrane with a top cut-off limit below 35 000 Dalton may be appropriate.

The solution containing the carrier with bound ligand reformed after the splitting off is reused preferably for contact with the original solution containing the peptide-containing compound as well as impurities, as a result of which a substantially closed circuit for the carrier with bound ligand is created. In certain cases it can be appropriate to add a buffer or the like for the reconditioning (pH-adjustment) of the solution containing the carrier with bound ligand prior to reuse.

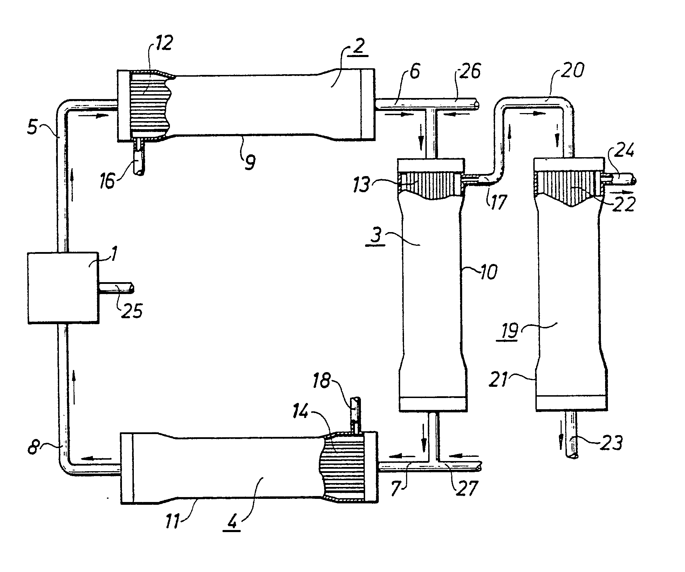

The invention will be described in more detail in the following with reference to the attached drawing which shows schematically an arrangement for the realization of the process in accordance with a practical embodiment.

Best mode of carrying out the invention

In the example shown a substantially closed system for the ligand bound to a carrier is used. The system comprises in turn an appropriately designed mixing chamber 1, a first membrane filter 2, a second membrane filter 3, and a third membrane filter 4 connected with one another by means of ducts 5, 6, 7 and 8 respectively, as shown in the figure.

The membrane filters 1, 2 and 3 are constituted preferably of semipermeable hollow fibres 12, 13 and 14 respectively with their respective axial fibre hollows adapted to form flow ducts for the ligand bound in the carrier in the closed system, as will be described. Moreover, the membrane filters comprise outlets 16, 17 and 18 respectively for the filtrate including matters which can penetrate the pores in the hollow fibre walls.

The first membrane filter 2 thus comprises semipermeable hollow fibres 12 with the axial fibre hollows in connection with the ducts 5 and 6. Moreover the membrane filter 1 comprises an outlet 16 for the impurities filtered off.

The second membrane filter 3, too, comprises preferaly semi-permeable hollow fibres 13 with the axial fibre hollows in connection with the ducts 6 and 7. Moreover, the membrane filter comprises an outlet 17 for the peptide-containing compound separated.

The third membrane filter 4 comprises preferably semipermeable hollow fibres 14 in connection with the ducts 8 and 7. Moreover, it comprises an outlet 18 for the salts or the like filtered off.

As can be seen in the figure, the arrangement may also comprise a fourth membrane filter 19 in connection with the outlet 17 on the second membrane filter 3 via a duct 20. This fourth membrane filter comprises a housing 21 with semipermeable hollow fibres 22 arranged inside the housing, the axial fibre hollow being connected to the duct 20, and an outlet 23 for the purified peptide-containing compound. Moreover, the fourth membrane filter 19 comprises an outlet 24, e.g. for free ligand filtered off, as will be described in the following.

Finally, the arrangement comprises a duct 25 in connection with the mixing chamber 1 for the introduction of solution containing the peptide-containing compound together with impurities, a duct 26 in connection with the duct 6 for the feed of dissociation agent, e.g. competing free ligand, an acid solution or a salt solution, and a duct 27 in connection with the duct 7 for the feed, for example of the buffer for reconditioning the ligand bound in the carrier.

The process in accordance with the invention is realized in the following manner with the help of the arrangement shown.

The solution containing the peptide-containing compound together with impurities is introduced into the mixing chamber 1 via the duct 25 from a source for this solution, not shown on the drawing. In the mixing chamber 1 the solution is brought into contact with the ligand bound to the carrier circulating in the closed system described above, a complex consisting of carrier/ligand/peptide-containing compound being formed. From the mixing chamber 1 the solution containing the complex formed, together with the impurities is pumped into the first membrane filter 2 via the duct 5 where the impurities are filtered out through the pores in the semipermeable hollow fibres 12 and are discharged via the outlet 16. The solution containing the complex

formed so purified is pumped from the first membrane filter 2 into the second membrane filter 3 via the duct 6 together with dissociating agent which is introduced into the duct 6 via the duct 26. In the second membrane filter 3 a filtering off of the peptide-containing compound thus split off, together with dissociating agent, takes place through the pores in the semipermeable hollow fibres 13. The solution containing the freed ligand bound to the carrier is pumped from the second membrane filter 3 into the third membrane filter 4 via the duct 7 together with a buffer solution which is fed via the duct 27 in connection with duct 7. From the third membrane filter 4 the regenerated ligand bound to the carrier is then pumped into the mixing chamber 1 via the duct 8 for reuse. Any residues of dissociating agent of salts remaining are filtered off from the solution through the pores in the semipermeable hollow fibres 14 and discharge via the outlet 18.

The peptide-containing compound freed in the second membrane filter 3 together with any dissociating agent is withdrawn via the outlet 17 and introduced into the fourth membrane filter 19 via the duct 20 for separation of the said dissociating agent which is filtered through the pores in the semipermeable hollow fibres 22 and withdrawn through the outlet 24. The purified peptide-containing compound is withdrawn through the outlet 23.

Industrial applicability

The process in accordance with the invention can be utilized for the recovery of practically any type of peptide containing compound from a solution which also contains one or more other compounds of contaminating character, but is applicable particularly for the recovery of enzymes, lectins, antibodies, antigens and haptens in pure form. Moreover, the invention in its widest scope can be used also for the recovery of types of compounds other than those enumerated above including charged compounds of the ionic type.

**Claims**

1. A process for the recovery of a peptide-containing compound from a solution also containing one or more other compounds of contaminating character, this process comprising the steps of
   a) mixing a solution of the said peptide containing compound and the said compounds of contaminating character with a ligand bound to a carrier in order to selectively form a complex consisting of carrier/ligand/peptide containing compound,
   b) subjecting the reaction mixture of step (a) to a membrane filtration in order to separate the complex formed in step (a) from the contaminating compounds,
   c) splitting off the compound to be recovered from the said complex formed in step (a) and
   d) separating the compound from the ligand bound to the carrier by membrane-filtration,

characterized in that the carrier is constituted of a macro-molecular solid material and that the peptide-containing compound is separated by using a semipermeable membrane which is permeable for the peptide-containing compound, but impermeable for the macromolecular solid material with the bound ligand.

2. A process in accordance with claim 1, characterized in that the solid macromolecular material is selected among cells, agarose, starch, cellulose, chitin, gelatine, collagen and synthetic polymers of the latex and acrylic derivatives type, as well as inorganic material, for example ceramic material, porous siliceous material etc.

3. A process in accordance with claim 1 or 2, characterized in that the solid macromolecular material is used in the form of particles of a size of over 0.1 µm, for example 1—50 µm, preferably 2—10 µm.

4. A process in accordance with any one of the preceding claims, characterized in that the said ligand is selected among inhibitors, carbohydrate structures, antigens, antibodies, haptens, cofactors, C1Q, enzymes, nucleic acids, conglutinin and surface receptors, for examples bacterial, such as protein A and protein G.

5. A process in accordance with any one of the preceding claims, characterized in that the peptide-containing compound is separated from the complex formed through the addition of an acid solution, a salt solution or a ligand present in free form which is capable of binding to the peptide-containing compound with simultaneous splitting off the same from the ligand bound to the carrier.

6. A process in accordance with claim 5, characterized in that the ligand present in free form is selected among low-molecular sugars, e.g. mono- and disaccharides in the purification of lectins and antigens or parts thereof in the purification of antibodies.

7. A process in accordance with claim 5 or 6, characterized in that the peptide-containing compound is separated from the ligand present in free form by using a semipermeable membrane (22) which is permeable for the free ligand, but impermeable for the peptide-containing compound or vice versa.

8. A process in accordance with any one of claims 5—7, characterized in that the ligand bound to the carrier freed in this manner is reused for contact with the solution containing the peptide-containing compound in accordance with claim 1.

9. A process in accordance with claim 8, characterized in that the freed ligand bound to the carrier is reconditioned for reuse through the addition of a suitable buffer solution.

**Patentansprüche**

1. Verfahren zur Gewinnung einer Peptidbindungen enthaltenden Verbindung aus einer Lösung, die auch eine oder mehrere andere Verbindungen von verunreinigendem Charakter enthält, mit den Stufen, bei denen man
   a) eine Lösung der Peptidbindungen enthaltenden Verbindung und der Verbindungen verunreinigenden Charakters mit einem an einen Träger gebundenen Liganden vermischt, um selektiv einen Komplex zu bilden, der aus Träger/Ligand/Peptidbindungen enthaltender Verbindung besteht,

b) das Reaktionsgemisch der Stufe (a) einer Membranfiltration unterzieht, um den in der Stufe (a) gebildeten Komplex von den verunreinigenden Verbindungen abzutrennen,

c) die zu gewinnende Verbindung von dem in der Stufe (a) gebildeten Komplex abspaltet und

d) die Verbindung von dem an den Träger gebundenen Liganden durch Membranfiltration abtrennt,

dadurch gekennzeichnet, daß der Träger aus einem makromolecularen festen Material besteht und daß die Peptidbindungen enthaltende Verbindung durch Verwendung einer semipermeablen Membran abgetrennt wird, die für die Peptidbindungen enthaltende Verbindung durchlässig, aber für das makromolekulare feste Material mit dem gebundenen Liganden undurchlässig ist.

2. Verfahren nach Anspruch 1, dadurch gekennzeichnet, daß das feste makromolekulare Material unter Zellen, Agarose, Stärke, Cellulose, Chitin, Gelatine, Collagen und synthetischen Polymeren vom Latex- und Acrylsäurderivattyp sowie unter anorganischen Materialien, wie beispielsweise Keramikmaterial, porösem kieselsäurehaltigem Material usw. ausgewählt ist.

3. Verfahren nach Anspruch 1 oder 2, dadurch gekennzeichnet, daß das feste makromolekulare Material in der Form von Teilchen einer Größe über 0,1 µm, wie beispielsweise 1 bis 50 µm, vorzugsweise 2 bis 10 µm, verwendet wird.

4. Verfahren nach einem der vorausgehenden Ansprüche, dadurch gekennzeichnet, daß der Ligand unter Inhitiboren, Kohlenhydratstrukturen, Antigenen, Antikörpern, Haptenen, Cofaktoren, $C_1Q$, Enzymen, Nucleinsäuren, Conglutinin und Oberflächenrezeptoren, wie beispielsweise bakteriellen, wie Protein A und Protein G, ausgewählt ist.

5. Verfahren nach einem der vorausgehenden Ansprüche, dadurch gekennzeichnet, daß die Peptidbindungen enthaltenden Verbindung von dem gebildeten Komplex durch die Zugabe einer Säurelösung, einer Salzlösung oder eines in freier Form vorliegenden Liganden, der in der Lage ist, sich unter gleichzeitiger Abspaltung der Peptidbindungen enthaltenden Verbindung von dem an den Träger gebundenen Liganden an diese Peptidbindungen enthaltende Verbindung zu binden, abgetrennt wird.

6. Verfahren nach Anspruch 5, dadurch gekennzeichnet, daß der in freier Form vorliegende Ligand unter niedermolekularen Zuckern, z.B. Mono- und Disacchariden, bei der Reinigung von Lectinen und Antigenen, oder von Teilen hiervon bei der Reinigung von Antikörpern ausgewählt ist.

7. Verfahren nach Anspruch 5 oder 6, dadurch gekennzeichnet, daß die Peptidbindungen enthaltende Verbindung von dem in freier Form vorliegenden Liganden durch Verwendung einer semipermeablen Membran (22) abgetrennt wird, die für den freien Liganden durchlässig, aber für die Peptidbindungen enthaltende Verbindung undurchlässig ist oder umgekehrt.

8. Verfahren nach einem der Ansprüche 5 bis 7, dadurch gekennzeichnet, daß der auf diese Weise freigesetzte, an den Träger gebundene Ligand zur Behandlung mit der Lösung, die Peptidbindungen enthaltende Verbindung enthält, gemäß Anspruch 1 wiederverwendet wird.

9. Verfahren nach Anspruch 8, dadurch gekennzeichnet, daß der freigesetzte, an den Träger gebundene Ligand für die Wiederverwendung durch die Zugabe einer geeigneten Pufferlösung rekonditioniert wird.

**Revendications**

1. Procédé pour la récupération d'un composé peptidique à partir d'une solution contenant également un ou plusieurs autres composés de caractère contaminant, ledit procédé comprenant les stades qui consistent à

a) mélanger une solution dudit composé peptidique et desdits composés de caractère contaminant avec un ligand fixé à un support pour former sélectivement un complexe support/ligand/composé peptidique,

b) soumettre le mélange réactionnel du stade (a) à une filtration sur membrane pour séparer le complexe formé dans le stade (a) des composés contaminants,

c) cliver le composé à récupérer dudit complexe formé lors du stade (a), et

d) séparer le composé du ligand fixé au support par filtration sur membrane, étant

caractérisé en ce que le support est constitué d'une matière solide macromoléculaire et en ce que le composé peptidique est séparé par utilisation d'une membrane semi-perméable qui est perméable au composé peptidique, mais imperméable à la matière solide macromoléculaire avec le ligand fixé.

2. Procédé selon la revendication 1, caractérisé en ce que la matière macromoléculaire solide est choisie parmi les cellules, l'agarose, l'amidon, la cellulose, la chitine, la gélatine, le collagène et les polymères synthétiques de type latex et dérivés acryliques ainsi qu'une matière minérale, notamment une matière céramique, une matière siliceuse poreuse, etc.

3. Procédé selon la revendication 1 ou 2, caractérisé en ce que la matière macromoléculaire solide est utilisée sous forme de particules ayant une taille supérieure à 0,1 µm, notamment de 1—50 µm et de préférence de 2—10 µm.

4. Procédé selon l'une quelconque des revendications précédentes, caractérisé en ce que ledit ligand est choisi parmi les inhibiteurs, les structures carbohydratées, les antigènes, les anticorps, les haptènes, les cofacteurs, le facteur C1Q, les enzymes, les acides nucléiques, la conglutinine et les récepteurs de surface, notamment bactériens, comme la protéine A et la protéine G.

5. Procédé selon l'une quelconque des revendications précédentes, caractérisé en ce que le composé peptidique est séparé du complexe, qui a été formé, par addition d'une solution d'un acide, d'une solution d'un sel ou d'un ligand présent sous une forme libre capable de se fixer au composé peptidique avec clivage simultané de ce dernier d'avec le ligand fixé au support.

6. Procédé selon la revendication 5, caractérisé en ce que le ligand présent sous la forme libre est choisi parmi les sucres de bas poids moléculaire, par exemple les mono- et disaccharides dans la purification des lectines et des antigènes ou des parties de ceux-ci dans la purification des anticorps.

7. Procédé selon la revendication 5 ou 6, caractérisé en ce que le composé peptidique est séparé du ligand présent sous forme libre par utilisation d'une membrane semi-perméable (22) qui est perméable au ligand libre mais imperméable au composé peptidique ou vice versa.

8. Procédé selon l'une quelconque des revendications 5 à 7, caractérisé en ce que le ligand fixé au support ainsi libéré est réutilisé pour mise en contact avec la solution contenant le composé peptidique selon la revendication 1.

9. Procédé selon la revendication 8, caractérisé en ce que le ligand libre fixé au support est reconditionné pour réutilisation par addition d'une solution tampon appropriée.